# EUROPEAN PATENT SPECIFICATION

(11) **EP 3 873 894 B1**
(45) Date of publication and mention of the grant of the patent: **22.03.2023**
(21) Application number: 19800947.4
(22) Date of filing: 30.10.2019
(51) Int. Cl.: C07D 401/14, A61P 35/00

(54) **NOVEL SALT OF A BCL-2 INHIBITOR, RELATED CRYSTALLINE FORM, METHOD FOR PREPARING THE SAME AND PHARMACEUTICAL COMPOSITIONS CONTAINING THE SAME**
NEUARTIGE SALZE EINES BCL-2-INHIBITORS, ZUGEHÖRIGE KRISTALLINE FORMEN, VERFAHREN ZUR HERSTELLUNG DAVON UND PHARMAZEUTISCHE ZUSAMMENSETZUNGEN DAMIT
NOUVEAU SEL D'UN INHIBITEUR DE BCL-2, FORME CRISTALLINE APPARENTÉE, SON PROCÉDÉ DE PRÉPARATION ET COMPOSITIONS PHARMACEUTIQUES LE CONTENANT

(30) Priority: 31.10.2018 EP 18306430
(43) Date of publication of application: 08.09.2021
(73) Proprietor: Les Laboratoires Servier, 92284 Suresnes (FR); Vernalis (R&D) Limited, Cambridge CB21 6GB (GB)
(72) Inventor: LYNCH, Michael, 45140 Saint Jean de la Ruelle (FR); VILLARD, Frédéric, 45470 Loury (FR); MOUCHET, Patrick, 76760 Ectot-l'Auber (FR); TAULELLE, Pascal, 76600 Le Havre (FR); MASSON, Ludovic, 76170 Saint-Antoine-la-Fôret (FR)
(86) International application number: PCT/EP2019/079621
(87) International publication number: WO 2020/089281

(56) References cited:
- WO-A1-2015/011400

## Description

### FIELD OF THE INVENTION

The invention relates to a novel salt of 5-(5-chloro-2-{[(3S)-3-(morpholin-4-ylmethyl)-3,4-dihydroisoquinolin-2(1*H*)-yl]carbonyl} phenyl)-*N*-(5-cyano-1,2-dimethyl-1*H*-pyrrol-3-yl)-*N-*(4-hydroxyphenyl)-1,2-dimethyl-1*H*-pyrrole-3-carboxamide, referred to herein as 'Compound A', or polymorphs or solvates thereof, methods for preparing the same as well as pharmaceutical compositions thereof. In particular, the invention relates to the hydrogen sulfate salt of Compound A, referred to herein as 'Compound A, H₂SO₄', and the crystalline form I thereof. The present invention further discloses a process for preparing said crystalline form and pharmaceutical compositions comprising said crystalline form. The invention also relates to the use of such compositions for the treatment of cancer, diseases of the immune system and auto-immune diseases. Last, an anhydrous crystalline form of Compound A, H₂SO₄ is disclosed.

### BACKGROUND OF THE INVENTION

The chemical structure of Compound A is:

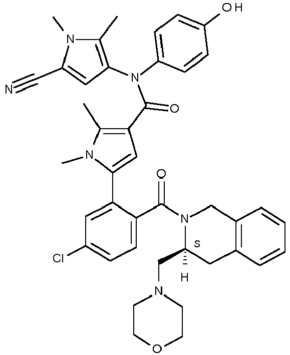

Its preparation, its use as a Bcl-2 inhibitor for the treatment of cancer and pharmaceutical formulations thereof are described in WO 2015/011400 (Example 386). The preparation of Compound A in the form of a hydrochloride salt ('Compound A.HCl') is specifically disclosed in this document. It is obtained as a lyophilisate.

Although Compound A is a very promising drug, it is a difficult compound to formulate. In particular, it is slightly soluble in water (< 0.01 mg/mL for the free base). As a chemical substance can exhibit different physical properties being in one or another salt form or crystalline form thereof, this polymorphism of the drug molecule can affect the shelf life, solubility, formulation properties, processing properties, and the action of a drug. In addition, different polymorphs can have different rates of uptake in the body, leading to lower or higher biological activity than desired. In extreme cases, an undesired polymorph can even show toxicity. Understanding and controlling polymorphism, then, gives a decided advantage in bringing new drugs to the marketplace, which may be more active, more stable, or more cheaply manufactured. However, even though polymorphism has been a subject for intensive investigations, understanding and controlling this phenomenon represents a substantial scientific challenge. It is hard to predict whether a given molecule will crystallize in one or several crystal forms, and to find conditions leading to such crystallization.

From the industrial point of view, it is imperative to be able to synthesise the compound with excellent purity, and in particular in a highly reproducible form, having valuable characteristics of dissolution, filtration, drying, ease of formulation and stability enabling the prolonged storage thereof without particular requirements for temperature, light, humidity or oxygen levels.

The present invention also describes a process for obtaining Compound A, H₂SO₄ in a well-defined, perfectly reproducible crystalline form (Form I) having very good stability that is compatible with the industrial constraints of preparation, especially filtration, and storage of pharmaceutical compositions.

### BRIEF DESCRIPTION OF THE FIGURES

Figure 1 shows the X-ray powder diffraction pattern (XPRD) of the crystalline form I of Compound A, H₂SO₄.
Figure 2 shows the X-ray powder diffraction pattern (XPRD) of the anhydrous crystalline form of Compound A, hydrogen sulfate salt.
Figure 3 shows the X-ray powder diffraction pattern (XPRD) of the crystalline form I of Compound A, hydrochloride salt
Figure 4 shows the DSC and TGA profiles of the crystalline form I of Compound A, hydrogen sulfate salt
Figure 5 shows the DSC and TGA profiles of the crystalline form I of Compound A, hydrochloride salt
Figure 6 shows the solid-state ¹³C NMR spectrum of the crystalline form I of Compound A, H₂SO₄.

### DETAILED DESCRIPTION OF THE INVENTION

As used herein, the term 'comprising' means 'including', and is not intended to exclude the presence of any additional component, unless the context suggests otherwise, for example when the components together sum to 100%.

The term "alcohols" means C₁-C₆ alcohols such as methanol, ethanol, n-propanol, isopropanol, n-butanol, isobutanol, pentanol, 2-pentanol, 3-pentanol, isopentanol, hexanol.

'Cancer' means a class of disease in which a group of cells display uncontrolled growth. Cancer types include haematological cancers (lymphoma and leukemia) and solid tumors including carcinoma, sarcoma, or blastoma. 'Cancer' includes cancer of the bladder, brain, breast and uterus, chronic lymphoid leukaemias, colorectal cancer, cancers of the oesophagus and liver, lymphoblastic leukaemias, acute myeloid leukaemia, lymphomas, for example non-Hodgkin's B-cell lymphoma and diffuse large B-cell lymphoma, melanomas, malignant haemopathies, for example myelodysplastic syndrome, myelomas, for example multiple myeloma, ovarian cancer, non-small-cell lung cancer, prostate cancer, pancreatic cancer and small-cell lung cancer.
'free molecule' and 'free base' are used interchangeably herein and refer to Compound A when not in salt form.

Described below are a number of embodiments of the invention.

In one embodiment, the present invention relates to the hydrogen sulfate salt of 5-(5-chloro-2-{[(3*S*)-3-(morpholin-4-ylmethyl)-3,4-dihydroisoquinolin-2(1*H*)-yl]carbonyl} phenyl)-*N*-(5-cyano-1,2-dimethyl-1*H*-pyrrol-3-yl)-*N*-(4-hydroxyphenyl)-1,2-dimethyl-1*H-*pyrrole-3-carboxamide (Compound A, H₂SO₄).

In another embodiment, the present invention relates to a crystalline form I of the hydrogen sulfate salt of 5-(5-chloro-2-{[(3*S*)-3-(morpholin-4-ylmethyl)-3,4-dihydroisoquinolin-2(1*H*)-yl]carbonyl} phenyl)-*N*-(5-cyano-1,2-dimethyl-1*H*-pyrrol-3-yl)-*N-*(4-hydroxyphenyl)-1,2-dimethyl-1*H*-pyrrole-3-carboxamide (Compound A, H₂SO₄), wherein the crystalline form has an X-ray powder diffraction diagram showing the following diffraction lines (Bragg's angle 2 theta, expressed in degrees ±0.2°): 5.55 ; 6.62 and 7.39.

In another embodiment, the present invention relates to a crystalline form I of the hydrogen sulfate salt of 5-(5-chloro-2-{[(3*S*)-3-(morpholin-4-ylmethyl)-3,4-dihydroisoquinolin-2(1*H*)-yl]carbonyl} phenyl)-*N*-(5-cyano-1,2-dimethyl-1*H*-pyrrol-3-yl)-*N-*(4-hydroxyphenyl)-1,2-dimethyl-1*H*-pyrrole-3-carboxamide (Compound A, H₂SO₄), wherein the crystalline form has an X-ray powder diffraction diagram showing at least 4, 5, 6, 7, 8, 9, 10, 11, 12, 13, 14 or all of the following diffraction lines (Bragg's angle 2 theta, expressed in degrees ±0.2°): 5.55; 5.62; 6.62; 7.39; 10.17; 11.49; 11.83; 16.01; 16.54; 17.04; 18.98; 19.18; 21.90; 22.28; 24.89.

In another embodiment, the present invention relates to the crystalline form I of the hydrogen sulfate salt of Compound A, characterized in that it has an X-ray powder diffraction diagram having the following diffraction lines (Bragg's angle 2 theta, expressed in degrees ±0.2°): 5.55; 5.62; 6.62; 7.39; 10.17; 11.49; 11.83; 16.01; 16.54; 17.04; 18.98; 19.18; 21.90; 22.28; 24.89.

In another embodiment, the present invention relates to the crystalline form I of the hydrogen sulfate salt of Compound A, characterized in that it has the following X-ray powder diffraction diagram, measured using a PANalytical X'Pert Pro MPD diffractometer with an X'Celerator detector and expressed in terms of line position (Bragg's angle 2 theta, expressed in degrees ±0.2°) and interplanar distances d (expressed in Å):

| **Line no.** | **Angle 2-theta (degrees)** | **Interplanar distance (Å)** |
|---|---|---|
| 1 | 5.55 | 15.93 |
| 2 | 5.62 | 15.73 |
| 3 | 6.62 | 13.36 |
| 4 | 7.39 | 11.95 |
| 5 | 10.17 | 8.70 |
| 6 | 11.49 | 7.70 |
| 7 | 11.83 | 7.48 |
| 8 | 16.01 | 5.53 |
| 9 | 16.54 | 5.36 |
| 10 | 17.04 | 5.20 |
| 11 | 18.98 | 4.67 |
| 12 | 19.18 | 4.63 |
| 13 | 21.90 | 4.06 |
| 14 | 22.28 | 3.99 |
| 15 | 24.89 | 3.58 |

In another embodiment, the present invention relates to the crystalline form I of the hydrogen sulfate salt of Compound A, characterised in that it has a solid-state ¹³C CP/MAS NMR spectrum having the following peaks (expressed in ppm ± 0.2 ppm): 173.31 ppm, 155.32 ppm, 140.46 ppm, 139.19 ppm, 137.42 ppm, 134.68 ppm, 131.65 ppm, 131.14 ppm, 129.37 ppm, 126.32 ppm, 118.77 ppm, 117.36 ppm, 116.54 ppm, 113.61 ppm, 112.69 ppm, 110.74 ppm, 102.33 ppm, 101.45 ppm, 63.06 ppm, 57.19 ppm, 54.87 ppm, 52.06 ppm, 44.71 ppm, 43.94 ppm, 34.42 ppm, 32.89 ppm, 31.28 ppm, 30.66 ppm, 14.40 ppm, 13.34 ppm, 12.49 ppm and 10.50 ppm.

In another embodiment, the present invention relates to a pharmaceutical composition comprising as active ingredient the hydrogen sulfate salt of Compound A in association with one or more pharmaceutically acceptable excipients.

In another embodiment, the present invention relates to a pharmaceutical composition comprising as active ingredient the crystalline form I of the hydrogen sulfate salt of Compound A in association with one or more pharmaceutically acceptable excipients.

In another embodiment, the present invention relates to a pharmaceutical composition comprising as active ingredient the hydrogen sulfate salt of Compound A or its crystalline form I for use in the treatment of cancers, auto-immune diseases and diseases of the immune system.

In another embodiment, the present invention relates to the hydrogen sulfate salt of Compound A or its crystalline form I for use as a medicament, preferably for use in the treatment of cancers, auto-immune diseases and diseases of the immune system.

In a preferred embodiment, the cancer is selected from the bladder, brain, breast and uterus cancers, chronic lymphoid leukaemias, colorectal cancer, cancers of the oesophagus and liver, lymphoblastic leukaemias, acute myeloid leukaemia, lymphomas, for example non-Hodgkin's B-cell lymphoma and diffuse large B-cell lymphoma, melanomas, malignant haemopathies, for example myelodysplastic syndrome, myelomas, for example multiple myeloma, ovarian cancer, non-small-cell lung cancer, prostate cancer, pancreatic cancer and small-cell lung cancer.

In another embodiment, the present invention relates to a process for the preparation of the crystalline form I of the hydrogen sulfate salt of Compound A, wherein the hydrogen sulfate salt of Compound A is crystallised in a polar medium.

In a preferred embodiment, the polar medium is composed of one or more solvents selected from water and alcohols.

In a preferred embodiment, the alcohol is ethanol.

In a preferred embodiment the polar medium is an ethanol/water mixture.

In another embodiment, the present invention relates to a process for the preparation of the crystalline form I of the hydrogen sulfate salt of Compound A, in which process the crystallisation is seeded using a very small amount of the crystalline form I of the hydrogen sulfate salt of Compound A.

In another embodiment, the present invention relates to an anhydrous crystalline form of the hydrogen sulfate salt of 5-(5-chloro-2-{[(3*S*)-3-(morpholin-4-ylmethyl)-3,4-dihydroisoquinolin-2(1*H*)-yl]carbonyl} phenyl)-*N*-(5-cyano-1,2-dimethyl-1*H*-pyrrol-3-yl)-*N-*(4-hydroxyphenyl)-1,2-dimethyl-1*H*-pyrrole-3-carboxamide (Compound A, H₂SO₄), wherein the crystalline form has an X-ray powder diffraction diagram showing at least 4, 5, 6, 7, 8, 9, 10, 11, 12, 13, 14, 15 or all of the following diffraction lines (Bragg's angle 2 theta, expressed in degrees ±0.2°): 5.19; 5.64; 6.74; 7.14; 8.04; 8.33; 9.17; 9.40; 10.68; 11.03; 11.35; 12.18; 12.59; 13.64; 14.78; 15.09.

In another embodiment, the present invention relates to an anhydrous crystalline form , characterized in that it has the following X-ray powder diffraction diagram, measured using a PANalytical X'Pert Pro MPD diffractometer with an X'Celerator detector and expressed in terms of line position (Bragg's angle 2 theta, expressed in degrees ±0.2°) and interplanar distances d (expressed in Å):

| **Line no.** | **Angle 2-theta (degrees)** | **Interplanar distance (Å)** |
|---|---|---|
| 1 | 5.19 | 17.03 |
| 2 | 5.64 | 15.66 |
| 3 | 6.74 | 13.12 |
| 4 | 7.14 | 12.39 |
| 5 | 8.04 | 10.99 |
| 6 | 8.33 | 10.61 |
| 7 | 9.17 | 9.64 |
| 8 | 9.40 | 9.41 |
| 9 | 10.68 | 8.29 |
| 10 | 11.03 | 8.02 |
| 11 | 11.35 | 7.79 |
| 12 | 12.18 | 7.26 |
| 13 | 12.59 | 7.03 |
| 14 | 13.64 | 6.49 |
| 15 | 14.78 | 5.99 |
| 16 | 15.09 | 5.87 |

Obtaining the crystalline form I of the hydrogen sulfate salt of Compound A has the advantage of having good characteristics of stability. More especially, only one crystalline form was observed in the range of solvents and temperatures used for the screening, showing a limited polymorphism of the hydrogen sulfate salt in the tested conditions. Furthermore, the crystalline form I of the hydrogen sulfate salt of Compound A thereby obtained is sufficiently stable to allow its storage for an extended period without particular conditions for temperature, light, humidity or oxygen levels.

The Examples herein below illustrate the invention but do not limit it in any way.

### Example 1: Process for obtaining crystalline form I of the hydrogen sulfate salt of Compound A

25 g of Compound A (free base) was placed in 239.5 g of ethanol at ambient temperature. The mixture was then heated at 65°C. A solution of sulphuric acid in water (4.27 g of H₂SO₄ + 59.87 g of water) was then added gradually. The mixture was stirred for 1 h before being cooled to 10°C. When the crystallisation was complete, the suspension was filtered, washed with an ethanol/water mixture à 10°C, filtered and dried under reduced pressure. After drying, crystalline form I of the hydrogen sulfate salt of Compound A was obtained in a yield of about 70% and with a purity greater than 99.8%. The solid was characterised by the X-ray powder as set out in Example 3.

In the crystallisation process according to the invention, Compound A (free base) is obtained by any process which may be used.

### Example 2: Process for obtaining crystalline form I of the hydrogen sulfate salt of Compound A (seeding)

25 g of Compound A (free base) was placed in 239.5 g of ethanol at ambient temperature. The mixture was then heated at 65°C. A solution of sulphuric acid in water (4.27 g of H₂SO₄ + 59.87 g of water) was then added gradually. The mixture was stirred for 30 minutes. The mixture was then cooled slightly before being seeded with the crystalline form I of the hydrogen sulfate salt of Compound A (2% by weight of starting material). The mixture was stirred for 30 minutes before being cooled to 10°C. When the crystallisation was complete, the suspension was filtered, washed with an ethanol/water mixture à 10°C, filtered and dried under reduced pressure. After drying, crystalline form I of the hydrogen sulfate salt of Compound A was obtained in a yield of about 70% and with a purity greater than 99.8%. The solid was characterised by the X-ray powder as set out in Example 3.

In the crystallisation process according to the invention, Compound A (free base) is obtained by any process which may be used.

### Example 3: Crystalline form I of the hydrogen sulfate salt of Compound A (X-ray powder diffraction diagram)

Recording of the data was carried out in the transmission mode using a PANalytical X'Pert Pro MPD diffractometer with an X'Celerator detector under the following conditions:
- Voltage 45 kV, current 40 mA,
- Mounting: theta/theta,
- Anode: copper,
- K alpha-1 wavelength: 1.54060 Å,
- K alpha-2 wavelength: 1.54443 Å,
- K alpha-2/K alpha-1 ratio: 0.5,
- Measurement mode: continuous from 3° to 55° (Bragg's angle 2 theta) in increments of 0.017°,
- Measurement time per step: 35.5301 s.

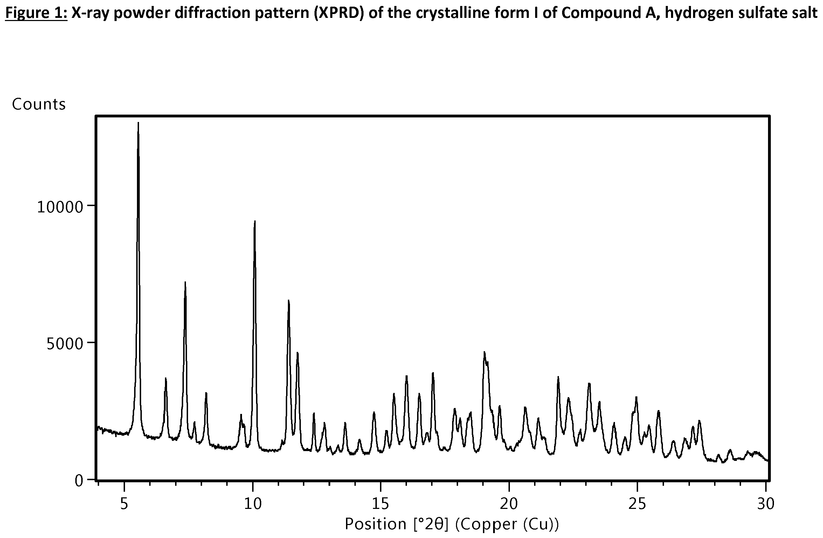

The X-ray powder diffraction diagram of the form I of the hydrogen sulfate salt of Compound A obtained according to the process of Example 1 or 2 is expressed in terms of line position (Bragg's angle 2 theta, expressed in degrees ±0.2°) and interplanar distances (expressed in Å) (Figure 1). The significant lines have been collated in the following table:

| **Line no.** | **Angle 2-theta (degrees)** | **Interplanar distance (Å)** |
|---|---|---|
| 1 | 5.55 | 15.93 |
| 2 | 5.62 | 15.73 |
| 3 | 6.62 | 13.36 |
| 4 | 7.39 | 11.95 |
| 5 | 10.17 | 8.70 |
| 6 | 11.49 | 7.70 |
| 7 | 11.83 | 7.48 |
| 8 | 16.01 | 5.53 |
| 9 | 16.54 | 5.36 |
| 10 | 17.04 | 5.20 |
| 11 | 18.98 | 4.67 |
| 12 | 19.18 | 4.63 |
| 13 | 21.90 | 4.06 |
| 14 | 22.28 | 3.99 |
| 15 | 24.89 | 3.58 |

### Example 4: Stability Studies

For all storage conditions and storage periods, 20 mg of crystalline form of the salt of Compound A were introduced in a 30-mL vial for post-storage HPLC analysis.

The drug substance content was determined by LC (% m/m).

| Temperature | Packaging | Hydrogen sulfate salt, crystalline form I |
|---|---|---|
| T₀ | | >99.9 |
| 25°C / 60°/RH | Double polyethylene bag placed in a plastic drum | 100.7 after 3 months of storage |
| 30°C / 65°/RH | Double polyethylene bag placed in a plastic drum | 100.6 after 3 months of storage |
| 40°C / 75°/RH | Double polyethylene bag placed in a plastic drum | 100.0 after 3 months of storage |
| 50°C / 75°/RH | Open glass bottle | 101.0 after 6 weeks of storage |

The appearance of the powder (white) and chemical stability remains unchanged under all conditions tested: over 3 months at 25°C/60%RH, 30°C/65%RH, 40°C/75%RH, and for 6 weeks at 50°C/75%RH.

Furthermore, the X-ray diffraction results show that the form does not change after analysis at T₀ and after 6 weeks storage in open glass bottles at 25°C/90%RH.

In conclusion, the drug substance can be considered physically and chemically stable over the periods tested.

### Example 5: Process for obtaining the anhydrous crystalline form of the hydrogen sulfate salt of Compound A (seeding)

5.83 kg of Compound A (free base) was placed in 55.85 kg of ethanol at ambient temperature. The mixture was then heated at 65°C. A solution of sulphuric acid in water (1 kg of H₂SO₄ + 13,96 kg of water) was then added gradually. The mixture was stirred for 30 minutes. The mixture was then cooled slightly before being seeded with the crystalline form I of the hydrogen sulfate salt of Compound A (2% by weight of starting material). The mixture was stirred for 30 minutes before being cooled to 10°C. When the crystallisation was complete, the suspension was filtered, washed with an ethanol/water mixture à 10°C, filtered and dried under reduced pressure. Then, the dried product is stored under an inert atmosphere (nitrogen). The anhydrous crystalline form of the hydrogen sulfate salt of Compound A was obtained in a yield of about 78 ± 5% and with a purity greater than 99.9% and a water content of about 0.43%. The solid was characterised by the X-ray powder as set out in Example 6.

In the crystallisation process according to the invention, Compound A (free base) is obtained by any process which may be used.

### Example 6: Anhydrous crystalline form of the hydrogen sulfate salt of Compound A (X-ray powder diffraction diagram)

Recording of the data was carried out in the following conditions:
Approximately 30-50 mg of the sample to be analysed is placed between two polymeric films (Kapton^{®}) fixed in a sample holder disc. The X-ray diffraction pattern of the test sample is recorded from 3° 2theta to at least 40° 2-theta in 15 min using an X-ray diffractometer operating in the transmission mode with CuKα radiation (λ = 1.5418 Å) at 40kV and 30mA and with a step size ranging from 0.01 to 0.02° 2-theta. These settings may vary according to the diffractometer used.

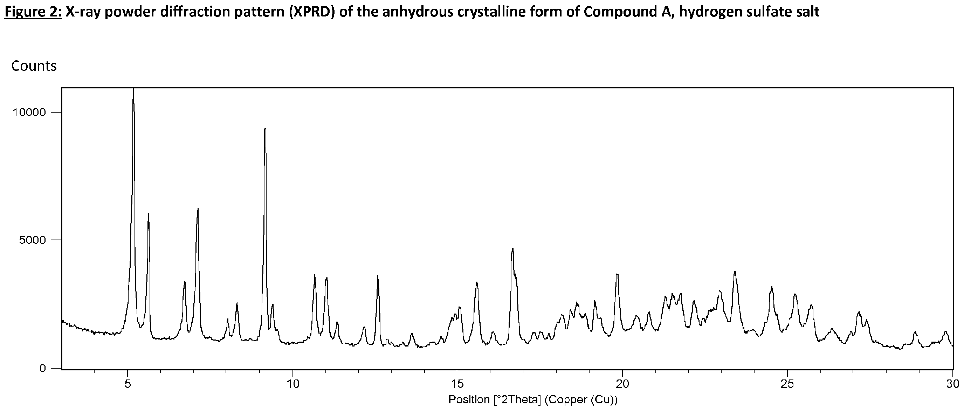

The X-ray powder diffraction diagram of the anhydrous form of the hydrogen sulfate salt of Compound A obtained according to the process of Example 5 is expressed in terms of line position (Bragg's angle 2 theta, expressed in degrees ±0.2°) and interplanar distances (expressed in Å) (Figure 2). The significant lines have been collated in the following table:

| **Line no.** | **Angle 2-theta (degrees)** | **Interplanar distance (Å)** |
|---|---|---|
| 1 | 5.19 | 17.03 |
| 2 | 5.64 | 15.66 |
| 3 | 6.74 | 13.12 |
| 4 | 7.14 | 12.39 |
| 5 | 8.04 | 10.99 |
| 6 | 8.33 | 10.61 |
| 7 | 9.17 | 9.64 |
| 8 | 9.40 | 9.41 |
| 9 | 10.68 | 8.29 |
| 10 | 11.03 | 8.02 |
| 11 | 11.35 | 7.79 |
| 12 | 12.18 | 7.26 |
| 13 | 12.59 | 7.03 |
| 14 | 13.64 | 6.49 |
| 15 | 14.78 | 5.99 |
| 16 | 15.09 | 5.87 |

### Example 7: Process for obtaining crystalline form I of the hydrogen sulfate salt of Compound A (seeding, batch size of the order of the kilogram)

5.83 kg of Compound A (free base) was placed in 55.85 kg of ethanol at ambient temperature. The mixture was then heated at 65°C. A solution of sulphuric acid in water (1 kg of H₂SO₄ + 13.96 kg of water) was then added gradually. The mixture was stirred for 30 minutes. The mixture was then cooled slightly before being seeded with the crystalline form I of the hydrogen sulfate salt of Compound A (2% by weight of starting material). The mixture was stirred for 30 minutes before being cooled to 10°C. When the crystallisation was complete, the suspension was filtered, washed with an ethanol/water mixture at 10°C, filtered and dried under reduced pressure. The product was rehydrated thereafter at 40°C under an atmosphere with 50% of relative humidity (RH). The resulting product was stored under an inert atmosphere (nitrogen). The crystalline form I of the hydrogen sulfate salt of Compound A was obtained in a yield of about 78 ± 5% and with a purity greater than 99.9% and a water content of about 6.5 ± 1%. The solid was characterised by the X-ray powder as set out in Example 3.

In the crystallisation process according to the invention, Compound A (free base) is obtained by any process which may be used.

### Example 8: Process for obtaining the crystalline form I of the hydrochloride salt of Compound A and the X-ray powder diffraction diagram characterising it

1510 mg of the amorphous hydrochloride salt of Compound A (Example 386 of WO 2015/011400) was converted into its crystalline ethanol solvate by slurrying in 15 mL of ethanol for 48h hours. The residual solid was filtered, washed twice with 1 mL of ethanol and then suspended in 10 mL of water for 5 min. After a difficult filtration, the residual solid was dried overnight at 30°C/10 mbar and analysed by X-ray diffraction (3-30° 2 theta/10 min).

The mode of preparation for the HCI salt is complicated by the fact that it initially results in an ethanol solvate which is replaced by H₂O after resuspension in water to give the hydrated form. The resulting hydrated HCI salt formed fine needles which were quite difficult to filter.

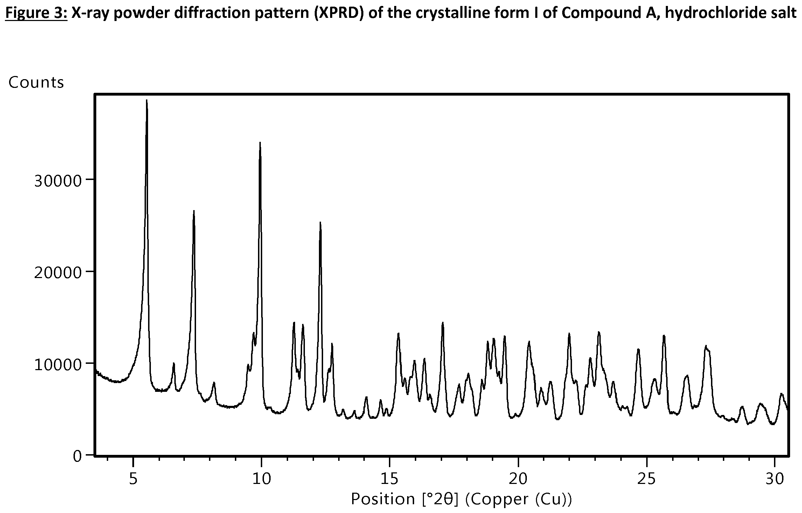

The X-ray powder diffraction diagram of the form I of the hydrochloride salt of Compound A obtained according to the process described previously is expressed in terms of line position (Bragg's angle 2 theta, expressed in degrees ±0.2°) and relative intensity (expressed in %) (Figure 3). The significant lines have been collated in the following table:

| **Line no.** | **Angle 2-theta (degrees)** | **Relative Intensity(%)** |
|---|---|---|
| 1 | 5.53 | 100.00 |
| 2 | 7.37 | 65.92 |
| 3 | 9.96 | 92.98 |
| 4 | 11.26 | 31.90 |
| 5 | 11.62 | 30.11 |
| 6 | 12.29 | 67.53 |
| 7 | 12.76 | 25.47 |
| 8 | 15.34 | 29.27 |
| 9 | 17.04 | 32.91 |
| 10 | 18.82 | 25.98 |
| 11 | 19.07 | 27.10 |
| 12 | 19.48 | 27.89 |
| 13 | 20.41 | 25.05 |
| 14 | 21.99 | 28.88 |
| 15 | 23.14 | 29.52 |
| 16 | 24.69 | 23.99 |
| 17 | 25.66 | 29.09 |
| 18 | 27.28 | 25.49 |

### Example 9: DSC and TGA profiles of the crystalline forms I of the hydrochloride and hydrogen sulfate salts of Compound A

### H₂SO₄ salt

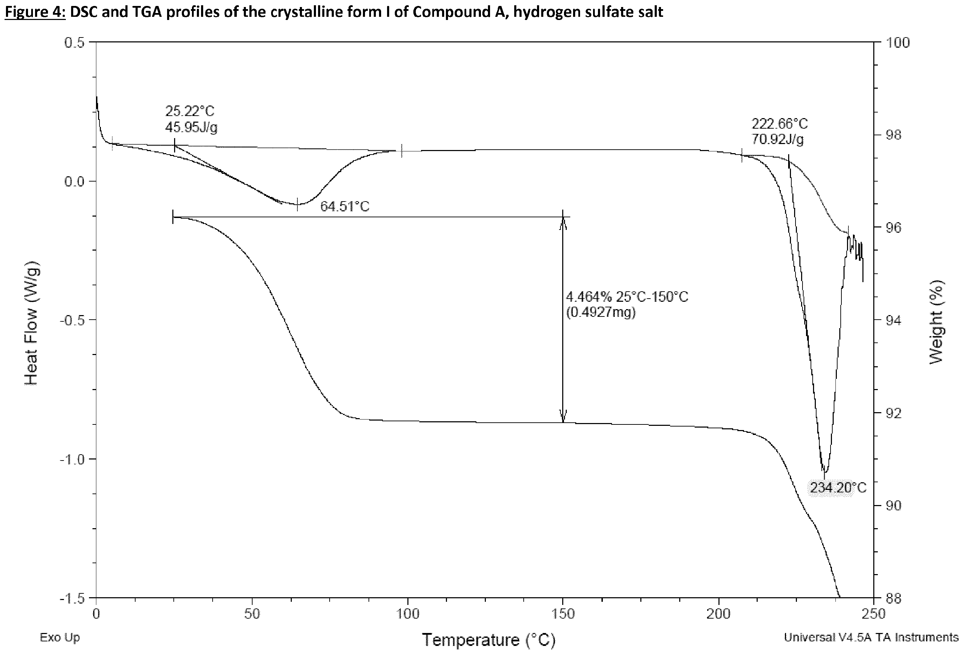

The Differential Scanning Calorimetry (DSC) profile of a sample of the hydrogensulfate salt, form I weighing approximately 4 mg was recorded between 0°C and 250°C at 10°C/min in pin-hole pierced aluminium pans under a positive flow of nitrogen on a TA Instruments Q1000 (or Q2000) Differential Scanning Calorimeter (Figure 4).

The Thermal Gravimetric Analysis (TGA) profile of a sample of the hydrogensulfate salt, form I weighing approximately 10 mg was recorded between 25°C and 250°C at 10°C/min in an open aluminium pan under a positive flow of nitrogen on a TA Instruments Q5000 Thermogravimetric Analyser (Figure 4).

### HCI salt

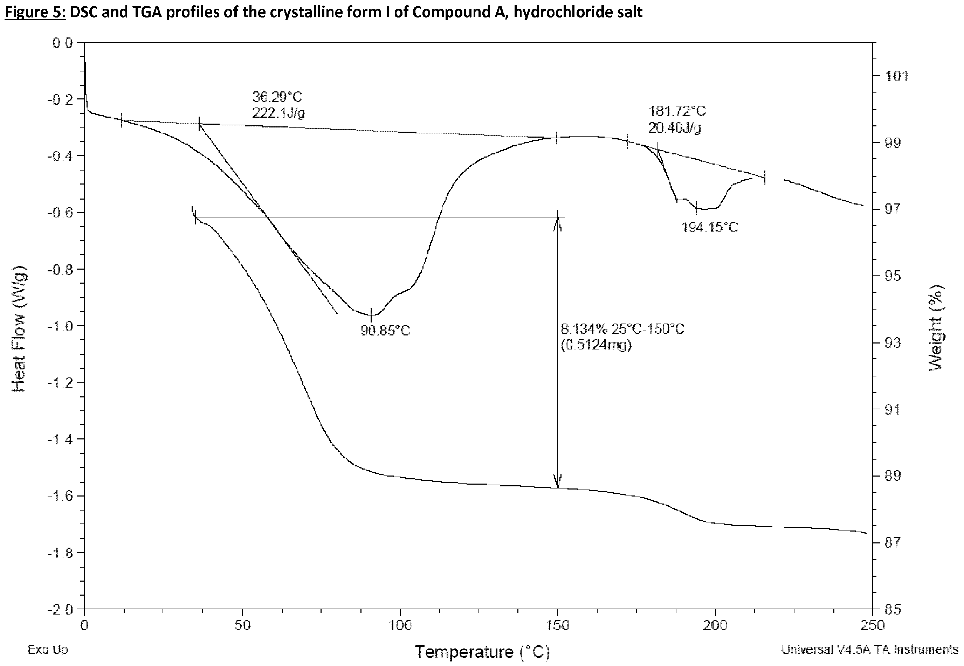

The DSC profile of a sample of the hydrochloride salt, form I weighing approximately 4 mg was recorded between 0°C and 250°C at 10°C/min in pin-hole pierced aluminium pans under a positive flow of nitrogen on a TA Instruments Q1000 (or Q2000) Differential Scanning Calorimeter (Figure 5).

The TGA profile of a sample of the hydrochloride salt, form I weighing approximately 6 mg was recorded between 25°C and 250°C at 10°C/min in an open aluminium pan under a positive flow of nitrogen on a TA Instruments Q5000 Thermogravimetric Analyser (Figure 5).

The DSC profile of the H₂SO₄ salt is less complicated compared to that of the HCI salt. Water loss is visible in the TGA profile of the H₂SO₄ salt between 25 and 100°C. A melting/degradation endotherm is visible in the DSC profile towards 224°C. The melting temperature and enthalpy of the HCI salt is lower than that of the H₂SO₄ salt. This may suggest that the HCI has a lower degree of crystallinity following dehydration compared to the H₂SO₄ salt.

### Example 10: Crystalline form I of Compound A, H₂SO₄(solid NMR Spectrum)

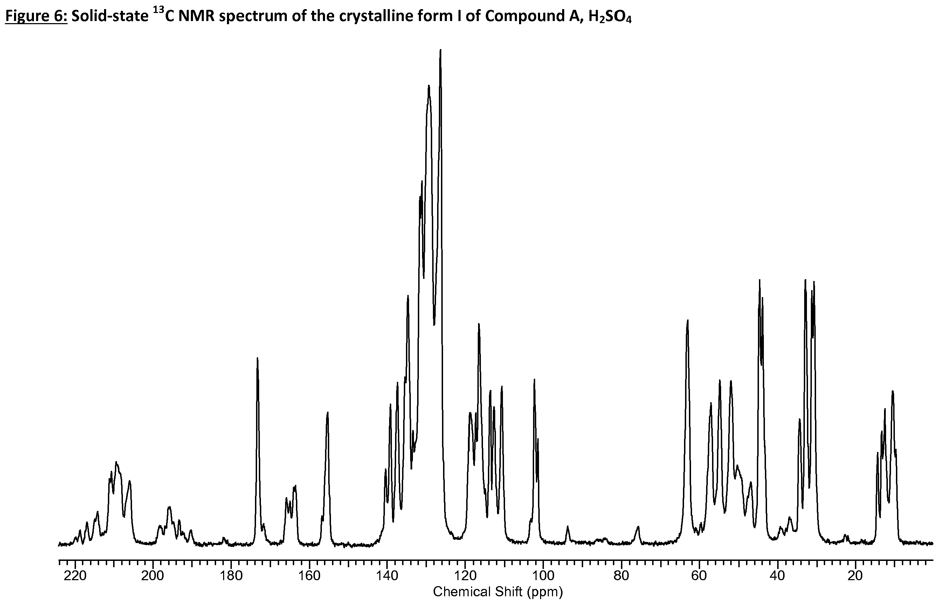

Crystalline form I of Compound A, H₂SO₄ was also characterized by solid-state Nuclear Magnetic Resonance spectroscopy (Figure 6). Solid-state ¹³C NMR spectra of Compound A, H₂SO₄ were recorded at ambient temperature using a Bruker SB Avance III HD 500 spectrometer with a 4 mm CP/MAS SB VTN type probe under the following conditions:
- Frequency: 125.76 MHz,
- Spectral width: 37 kHz,
- Magic angle spinning rate: 10 kHz,
- Pulse program: Cross Polarization with SPINAL64 decoupling
- Recycle delay: 10 s,
- Acquisition time: 46 ms,
- Contact time: 4 ms,
- Number of scans: 4096.

A 5 Hz line-broadening was applied prior to Fourier Transformation.

The spectrum thereby obtained was referenced relative to a sample of adamantane (the high frequency peak of adamantane was set to 38.5 ppm).

Crystalline form I of Compound A, H₂SO₄ can be defined by the presence of a set of peaks whose chemical shifts are given in the table below (expressed in ppm ± 0.2 ppm):

| No. | (ppm) |
|---|---|
| 1 | 173.31 |
| 2 | 155.32 |
| 3 | 140.46 |
| 4 | 139.19 |
| 5 | 137.42 |
| 6 | 134.68 |
| 7 | 131.65 |
| 8 | 131.14 |
| 9 | 129.37 |
| 10 | 126.32 |
| 11 | 118.77 |
| 12 | 117.36 |
| 13 | 116.54 |
| 14 | 113.61 |
| 15 | 112.69 |
| 16 | 110.74 |
| 17 | 102.33 |
| 18 | 101.45 |
| 19 | 63.06 |
| 20 | 57.19 |
| 21 | 54.87 |
| 22 | 52.06 |
| 23 | 44.71 |
| 24 | 43.94 |
| 25 | 34.42 |
| 26 | 32.89 |
| 27 | 31.28 |
| 28 | 30.66 |
| 29 | 14.40 |
| 30 | 13.34 |
| 31 | 12.49 |
| 32 | 10.50 |

## Claims

1. The hydrogen sulfate salt of 5-(5-chloro-2-{[(3*S*)-3-(morpholin-4-ylmethyl)-3,4-dihydroisoquinolin-2(1*H*)-yl]carbonyl} phenyl)-*N*-(5-cyano-1,2-dimethyl-1*H*-pyrrol-3-yl)-*N-*(4-hydroxyphenyl)-1,2-dimethyl-1*H*-pyrrole-3-carboxamide (Compound A, H₂SO₄).

2. A crystalline form I of the hydrogen sulfate salt of 5-(5-chloro-2-{[(3*S*)-3-(morpholin-4-ylmethyl)-3,4-dihydroisoquinolin-2(1*H*)-yl]carbonyl}phenyl)-*N*-(5-cyano-1,2-dimethyl-1*H*-pyrrol-3-yl)-*N*-(4-hydroxyphenyl)-1,2-dimethyl-1*H*-pyrrole-3-carboxamide (Compound A, H₂SO₄) according to claim 1, wherein the crystalline form has an X-ray powder diffraction diagram showing the following diffraction lines (Bragg's angle 2 theta, expressed in degrees ±0.2°): 5.55 ; 6.62 and 7.39.

3. A crystalline form I of the hydrogen sulfate salt of 5-(5-chloro-2-{[(3*S*)-3-(morpholin-4-ylmethyl)-3,4-dihydroisoquinolin-2(1*H*)-yl]carbonyl} phenyl)-*N*-(5-cyano-1,2-dimethyl-1*H*-pyrrol-3-yl)-*N*-(4-hydroxyphenyl)-1,2-dimethyl-1*H*-pyrrole-3-carboxamide (Compound A, H₂SO₄) according to claim 1, wherein the crystalline form has an X-ray powder diffraction diagram showing at least 4, 5, 6, 7, 8, 9, 10, 11, 12, 13, 14 or all of the following diffraction lines (Bragg's angle 2 theta, expressed in degrees ±0.2°): 5.55; 5.62; 6.62; 7.39; 10.17; 11.49; 11.83; 16.01; 16.54; 17.04; 18.98; 19.18; 21.90; 22.28; 24.89.

4. The crystalline form I of the hydrogen sulfate salt of Compound A according to claim 3, **characterized in that** it has an X-ray powder diffraction diagram having the following diffraction lines (Bragg's angle 2 theta, expressed in degrees ±0.2°): 5.55; 5.62; 6.62; 7.39; 10.17; 11.49; 11.83; 16.01; 16.54; 17.04; 18.98; 19.18; 21.90; 22.28; 24.89.

5. The crystalline form I of the hydrogen sulfate salt of Compound A according to claim 4, **characterized in that** it has the following X-ray powder diffraction diagram, measured using a PANalytical X'Pert Pro MPD diffractometer with an X'Celerator detector and expressed in terms of line position (Bragg's angle 2 theta, expressed in degrees ±0.2°) and interplanar distances d (expressed in Å):
| **Line no.** | **Angle 2-theta (degrees)** | **Interplanar distance (Å)** |
|---|---|---|
| 1 | 5.55 | 15.93 |
| 2 | 5.62 | 15.73 |
| 3 | 6.62 | 13.36 |
| 4 | 7.39 | 11.95 |
| 5 | 10.17 | 8.70 |
| 6 | 11.49 | 7.70 |
| 7 | 11.83 | 7.48 |
| 8 | 16.01 | 5.53 |
| 9 | 16.54 | 5.36 |
| 10 | 17.04 | 5.20 |
| 11 | 18.98 | 4.67 |
| 12 | 19.18 | 4.63 |
| 13 | 21.90 | 4.06 |
| 14 | 22.28 | 3.99 |
| 15 | 24.89 | 3.58 |

6. The crystalline form I of the hydrogen sulfate salt of Compound A according to any one of claims 1 to 5, **characterised in that** it has a solid-state ¹³C CP/MAS NMR spectrum having the following peaks (expressed in ppm ± 0.2 ppm): 173.31 ppm, 155.32 ppm, 140.46 ppm, 139.19 ppm, 137.42 ppm, 134.68 ppm, 131.65 ppm, 131.14 ppm, 129.37 ppm, 126.32 ppm, 118.77 ppm, 117.36 ppm, 116.54 ppm, 113.61 ppm, 112.69 ppm, 110.74 ppm, 102.33 ppm, 101.45 ppm, 63.06 ppm, 57.19 ppm, 54.87 ppm, 52.06 ppm, 44.71 ppm, 43.94 ppm, 34.42 ppm, 32.89 ppm, 31.28 ppm, 30.66 ppm, 14.40 ppm, 13.34 ppm, 12.49 ppm and 10.50 ppm.

7. Pharmaceutical composition comprising as active ingredient the hydrogen sulfate salt of Compound A according to claim 1 in association with one or more pharmaceutically acceptable excipients.

8. Pharmaceutical composition comprising as active ingredient the crystalline form I of the hydrogen sulfate salt of Compound A according to any one of claims 2 to 6 in association with one or more pharmaceutically acceptable excipients.

9. Pharmaceutical composition according to claim 7 or 8 for use in the treatment of cancers, auto-immune diseases and diseases of the immune system.

10. Pharmaceutical composition for use according to claim 9, wherein the cancer is selected from the bladder, brain, breast and uterus cancers, chronic lymphoid leukaemias, colorectal cancer, cancers of the oesophagus and liver, lymphoblastic leukaemias, acute myeloid leukaemia, lymphomas, for example non-Hodgkin's B-cell lymphoma and diffuse large B-cell lymphoma, melanomas, malignant haemopathies, for example myelodysplastic syndrome, myelomas, for example multiple myeloma, ovarian cancer, non-small-cell lung cancer, prostate cancer, pancreatic cancer and small-cell lung cancer.

11. The hydrogen sulfate salt of Compound A according to claim 1 for use as a medicament.

12. The crystalline form I of the hydrogen sulfate salt of Compound A according to any one of claims 2 to 6 for use as a medicament.

13. Process for the preparation of the crystalline form I of the hydrogen sulfate salt of Compound A according to any one of claims 2 to 6, wherein the hydrogen sulfate salt of Compound A is crystallised in a polar medium.

14. Process for the preparation of the crystalline form I of the hydrogen sulfate salt of Compound A according to claim 13, wherein the polar medium is composed of one or more solvents selected from water and alcohols.

15. Process for the preparation of the crystalline form I of the hydrogen sulfate salt of Compound A according to claim 14, wherein the alcohol is ethanol.

16. Process for the preparation of the crystalline form I of the hydrogen sulfate salt of Compound A according to claim 14, wherein the polar medium is an ethanol/water mixture.

17. Process for the preparation of the crystalline form I of the hydrogen sulfate salt of Compound A according to any one of claim 13 to 16, in which process the crystallisation is seeded using a very small amount of the crystalline form I of the hydrogen sulfate salt of Compound A.

18. Anhydrous crystalline form of the hydrogen sulfate salt of 5-(5-chloro-2-{[(3*S*)-3-(morpholin-4-ylmethyl)-3,4-dihydroisoquinolin-2(1*H*)-yl]carbonyl}phenyl)-*N*-(5-cyano-1,2-dimethyl-1*H*-pyrrol-3-yl)-*N*-(4-hydroxyphenyl)-1,2-dimethyl-1*H*-pyrrole-3-carboxamide (Compound A, H₂SO₄) according to claim 1, wherein the crystalline form has an X-ray powder diffraction diagram showing at least 4, 5, 6, 7, 8, 9, 10, 11, 12, 13, 14, 15 or all of the following diffraction lines (Bragg's angle 2 theta, expressed in degrees ±0.2°): 5.19; 5.64; 6.74; 7.14; 8.04; 8.33; 9.17; 9.40; 10.68; 11.03; 11.35; 12.18; 12.59; 13.64; 14.78; 15.09.

19. The anhydrous crystalline form of the hydrogen sulfate salt of Compound A according to claim 18, **characterized in that** it has the following X-ray powder diffraction diagram, measured using a PANalytical X'Pert Pro MPD diffractometer with an X'Celerator detector and expressed in terms of line position (Bragg's angle 2 theta, expressed in degrees ±0.2°) and interplanar distances d (expressed in Å):
| **Line no.** | **Angle 2-theta (degrees)** | **Interplanar distance (Å)** |
|---|---|---|
| 1 | 5.19 | 17.03 |
| 2 | 5.64 | 15.66 |
| 3 | 6.74 | 13.12 |
| 4 | 7.14 | 12.39 |
| 5 | 8.04 | 10.99 |
| 6 | 8.33 | 10.61 |
| 7 | 9.17 | 9.64 |
| 8 | 9.40 | 9.41 |
| 9 | 10.68 | 8.29 |
| 10 | 11.03 | 8.02 |
| 11 | 11.35 | 7.79 |
| 12 | 12.18 | 7.26 |
| 13 | 12.59 | 7.03 |
| 14 | 13.64 | 6.49 |
| 15 | 14.78 | 5.99 |
| 16 | 15.09 | 5.87 |

## Patentansprüche

1. Hydrogensulfatsalz von 5-(5-Chlor-2-{[(3*S*)-3-(morpholin-4-ylmethyl)-3,4-dihydroisochinolin-2(1*H*)-yl]carbonyl}phenyl)-*N*-(5-cyano-1,2-dimethyl-1*H*-pyrrol-3-yl)-*N-*(4-hydroxyphenyl)-1,2-dimethyl-1*H*-pyrrol-3-carboxamid (Verbindung A, H₂SO₄).

2. Kristalline Form I des Hydrogensulfatsalzes von 5-(5-Chlor-2-{[(3*S*)-3-(morpholin-4-ylmethyl)-3,4-dihydroisochinolin-2(1*H*)-yl]carbonyl}phenyl)-*N*-(5-cyano-1,2-dimethyl-1*H*-pyrrol-3-yl)-*N*-(4-hydroxyphenyl)-1,2-dimethyl-1*H*-pyrrol-3-carboxamid (Verbindung A, H₂SO₄) nach Anspruch 1, wobei die kristalline Form ein Pulver-Röntgenbeugungsdiagramm aufweist, das die folgenden Beugungslinien zeigt (Bragg-Winkel 2 Theta, ausgedrückt in Grad ±0,2°): 5,55; 6,62 und 7,39.

3. Kristalline Form I des Hydrogensulfatsalzes von 5-(5-Chlor-2-{[(3*S*)-3-(morpholin-4-ylmethyl)-3,4-dihydroisochinolin-2(1*H*)-yl]carbonyl}phenyl)-*N*-(5-cyano-1,2-dimethyl-1*H*-pyrrol-3-yl)-*N*-(4-hydroxyphenyl)-1,2-dimethyl-1*H*-pyrrol-3-carboxamid (Verbindung A, H₂SO₄) nach Anspruch 1, wobei die kristalline Form ein Pulver-Röntgenbeugungsdiagramm aufweist, das mindestens 4, 5, 6, 7, 8, 9, 10, 11, 12, 13, 14 oder alle der folgenden Beugungslinien zeigt (Bragg-Winkel 2 Theta, ausgedrückt in Grad ±0,2°): 5,55; 5,62; 6,62; 7,39; 10,17; 11,49; 11,83; 16,01; 16,54; 17,04; 18,98; 19,18; 21,90; 22,28; 24,89.

4. Kristalline Form I des Hydrogensulfatsalzes von Verbindung A nach Anspruch 3, **dadurch gekennzeichnet, dass** sie ein Pulver-Röntgenbeugungsdiagramm mit den folgenden Beugungslinien aufweist (Bragg-Winkel 2 Theta, ausgedrückt in Grad ±0,2°): 5,55; 5,62; 6,62; 7,39; 10,17; 11,49; 11,83; 16,01; 16,54; 17,04; 18,98; 19,18; 21,90; 22,28; 24,89.

5. Kristalline Form I des Hydrogensulfatsalzes von Verbindung A nach Anspruch 4, **dadurch gekennzeichnet, dass** sie das folgende Pulver-Röntgenbeugungsdiagramm aufweist, gemessen unter Verwendung eines PANalytical X'Pert Pro MPD-Diffraktometers mit einem X'Celerator-Detektor und ausgedrückt als Linienposition (Bragg-Winkel 2 Theta, ausgedrückt in Grad ±0,2°) und interplanare Abstände d (ausgedrückt in Ä):
| Liniennr. | Winkel 2 Theta (Grad) | Interplanarer Abstand (Å) |
|---|---|---|
| 1 | 5,55 | 15,93 |
| 2 | 5,62 | 15,73 |
| 3 | 6,62 | 13,36 |
| 4 | 7,39 | 11,95 |
| 5 | 10,17 | 8,70 |
| 6 | 11,49 | 7,70 |
| 7 | 11,83 | 7,48 |
| 8 | 16,01 | 5,53 |
| 9 | 16,54 | 5,36 |
| 10 | 17,04 | 5,20 |
| 11 | 18,98 | 4,67 |
| 12 | 19,18 | 4,63 |
| 13 | 21,90 | 4,06 |
| 14 | 22,28 | 3,99 |
| 15 | 24,89 | 3,58 |

6. Kristalline Form I des Hydrogensulfatsalzes von Verbindung A nach einem der Ansprüche 1 bis 5, **dadurch gekennzeichnet, dass** sie ein ¹³C-CP/MAS-Festkörper-NMR-Spektrum mit den folgenden Peaks (ausgedrückt in ppm ± 0,2 ppm) aufweist: 173,31 ppm, 155,32 ppm, 140,46 ppm, 139,19 ppm, 137,42 ppm, 134,68 ppm, 131,65 ppm, 131,14 ppm, 129,37 ppm, 126,32 ppm, 118,77 ppm, 117,36 ppm, 116,54 ppm, 113,61 ppm, 112,69 ppm, 110,74 ppm, 102,33 ppm, 101,45 ppm, 63,06 ppm, 57,19 ppm, 54,87 ppm, 52,06 ppm, 44,71 ppm, 43,94 ppm, 34,42 ppm, 32,89 ppm, 31,28 ppm, 30,66 ppm, 14,40 ppm, 13,34 ppm, 12,49 ppm und 10,50 ppm.

7. Pharmazeutische Zusammensetzung, umfassend als Wirkstoff das Hydrogensulfatsalz von Verbindung A nach Anspruch 1 in Verbindung mit einem oder mehreren pharmazeutisch verträglichen Hilfsstoffen.

8. Pharmazeutische Zusammensetzung, umfassend als Wirkstoff die kristalline Form I des Hydrogensulfatsalzes von Verbindung A nach einem der Ansprüche 2 bis 6 in Verbindung mit einem oder mehreren pharmazeutisch verträglichen Hilfsstoffen.

9. Pharmazeutische Zusammensetzung nach Anspruch 7 oder 8 zur Verwendung bei der Behandlung von Krebs, Autoimmunerkrankungen und Erkrankungen des Immunsystems.

10. Pharmazeutische Zusammensetzung zur Verwendung nach Anspruch 9, wobei der Krebs ausgewählt ist aus Blasen-, Hirn-, Brust- und Gebärmutterkrebs, chronisch lymphatischen Leukämien, kolorektalem Krebs, Speiseröhren- und Leberkrebs, lymphoblastischen Leukämien, akuter myeloischer Leukämie, Lymphomen, beispielsweise Non-Hodgkin-B-Zell-Lymphom und diffuses großzelliges B-Zell-Lymphom, Melanomen, malignen Hämopathien, beispielsweise myelodysplastisches Syndrom, Myelomen, beispielsweise multiples Myelom, Eierstockkrebs, nichtkleinzelligem Lungenkrebs, Prostatakrebs, Bauchspeicheldrüsenkrebs und kleinzelligem Lungenkrebs.

11. Hydrogensulfatsalz von Verbindung A nach Anspruch 1 zur Verwendung als Medikament.

12. Kristalline Form I des Hydrogensulfatsalzes von Verbindung A nach einem der Ansprüche 2 bis 6 zur Verwendung als Medikament.

13. Verfahren zur Herstellung der kristallinen Form I des Hydrogensulfatsalzes von Verbindung A nach einem der Ansprüche 2 bis 6, wobei das Hydrogensulfatsalz von Verbindung A in einem polaren Medium kristallisiert wird.

14. Verfahren zur Herstellung der kristallinen Form I des Hydrogensulfatsalzes von Verbindung A nach Anspruch 13, wobei das polare Medium aus einem oder mehreren Lösungsmitteln, ausgewählt aus Wasser und Alkoholen, zusammengesetzt ist.

15. Verfahren zur Herstellung der kristallinen Form I des Hydrogensulfatsalzes von Verbindung A nach Anspruch 14, wobei der Alkohol Ethanol ist.

16. Verfahren zur Herstellung der kristallinen Form I des Hydrogensulfatsalzes von Verbindung A nach Anspruch 14, wobei das polare Medium ein Ethanol/WasserGemisch ist.

17. Verfahren zur Herstellung der kristallinen Form I des Hydrogensulfatsalzes von Verbindung A nach einem der Ansprüche 13 bis 16, in welchem Verfahren die Kristallisation unter Verwendung einer sehr kleinen Menge der kristallinen Form I des Hydrogensulfatsalzes von Verbindung A angeimpft wird.

18. Wasserfreie kristalline Form des Hydrogensulfatsalzes von 5-(5-Chlor-2-{[(3*S*)-3-(morpholin-4-ylmethyl)-3,4-dihydroisochinolin-2(1*H*)-yl]carbonyl}phenyl)-*N*-(5-cyano-1,2-dimethyl-1*H*-pyrrol-3-yl)-*N-*(4-hydroxyphenyl)-1,2-dimethyl-1*H*-pyrrol-3-carboxamid (Verbindung A, H₂SO₄) nach Anspruch 1, wobei die kristalline Form ein Pulver-Röntgenbeugungsdiagramm aufweist, das mindestens 4, 5, 6, 7, 8, 9, 10, 11, 12, 13, 14, 15 oder alle der folgenden Beugungslinien zeigt (Bragg-Winkel 2 Theta, ausgedrückt in Grad ±0,2°): 5,19; 5,64; 6,74; 7,14; 8,04; 8,33; 9,17; 9,40; 10,68; 11,03; 11,35; 12,18; 12,59; 13,64; 14,78; 15,09.

19. Wasserfreie kristalline Form des Hydrogensulfatsalzes von Verbindung A nach Anspruch 18, **dadurch gekennzeichnet, dass** sie das folgende Pulver-Röntgenbeugungsdiagramm aufweist, gemessen unter Verwendung eines PANalytical X'Pert Pro MPD-Diffraktometers mit einem X'Celerator-Detektor und ausgedrückt als Linienposition (Bragg-Winkel 2 Theta, ausgedrückt in Grad ±0,2°) und interplanare Abstände d (ausgedrückt in Å):
| Liniennr. | Winkel 2 Theta (Grad) | Interplanarer Abstand (Å) |
|---|---|---|
| 1 | 5,19 | 17,03 |
| 2 | 5,64 | 15,66 |
| 3 | 6,74 | 13,12 |
| 4 | 7,14 | 12,39 |
| 5 | 8,04 | 10,99 |
| 6 | 8,33 | 10,61 |
| 7 | 9,17 | 9,64 |
| 8 | 9,40 | 9,41 |
| 9 | 10,68 | 8,29 |
| 10 | 11,03 | 8,02 |
| 11 | 11,35 | 7,79 |
| 12 | 12,18 | 7,26 |
| 13 | 12,59 | 7,03 |
| 14 | 13,64 | 6,49 |
| 15 | 14,78 | 5,99 |
| 16 | 15,09 | 5,87 |

## Revendications

1. Sel hydrogénosulfate de 5-(5-chloro-2-{[(3*S*)-3-(morpholin-4-ylméthyl)-3,4-dihydroisoquinolin-2(1*H*)-yl]carbonyl}phényl)-N-(5-cyano-1,2-diméthyl-1*H*-pyrrol-3-yl)-N-(4-hydroxyphényl)-1,2-diméthyl-1*H*-pyrrole-3-carboxamide (Composé A, H₂SO₄).

2. Forme cristalline I du sel hydrogénosulfate de 5-(5-chloro-2-{[(3*S*)-3-(morpholin-4-ylméthyl)-3,4-dihydroisoquinolin-2 (1*H*)-yl]carbonyl}phényl)-*N*-(5-cyano-1,2-diméthyl-1*H*-pyrrol-3-yl)-*N*-(4-hydroxyphényl)-1,2-diméthyl-1*H*-pyrrole-3-carboxamide (Composé A, H₂SO₄) selon la revendication 1, dans laquelle la forme cristalline présente un diagramme de diffraction des rayons X sur poudre présentant les raies de diffraction suivantes (angle de Bragg 2 thêta, exprimé en degrés ± 0,2°) : 5,55 ; 6,62 et 7,39.

3. Forme cristalline I du sel hydrogénosulfate de 5-(5-chloro-2-{[(3*S*)-3-(morpholin-4-ylméthyl)-3,4-dihydroisoquinolin-2(1*H*)-yl]carbonyl}phényl)-*N*-(5-cyano-1,2-diméthyl-1*H*-pyrrol-3-yl)-*N*-(4-hydroxyphényl)-1,2-diméthyl-1*H*-pyrrole-3-carboxamide (Composé A, H₂SO₄) selon la revendication 1, dans laquelle la forme cristalline présente un diagramme de diffraction des rayons X sur poudre présentant au moins 4, 5, 6, 7, 8, 9, 10, 11, 12, 13, 14 ou l'ensemble des raies de diffraction suivantes (angle de Bragg 2 thêta, exprimé en degrés ± 0,2°) : 5,55 ; 5,62 ; 6,62 ; 7,39 ; 10,17 ; 11,49 ; 11,83 ; 16,01 ; 16,54 ; 17,04 ; 18,98 ; 19,18 ; 21,90 ; 22,28 ; 24,89.

4. Forme cristalline I du sel hydrogénosulfate du Composé A selon la revendication 3, **caractérisée en ce qu'**elle présente un diagramme de diffraction des rayons X sur poudre ayant les raies de diffraction suivantes (angle de Bragg 2 thêta, exprimé en degrés ± 0,2°) : 5,55 ; 5,62 ; 6,62 ; 7,39 ; 10,17 ; 11,49 ; 11,83 ; 16,01 ; 16,54 ; 17,04 ; 18,98 ; 19,18 ; 21,90 ; 22,28 ; 24.89.

5. Forme cristalline I du sel hydrogénosulfate du Composé A selon la revendication 4, **caractérisée en ce qu'**elle présente le diagramme de diffraction des rayons X sur poudre suivant, mesuré à l'aide d'un diffractomètre PANalytical X'Pert Pro MPD avec un détecteur X'Celerator et exprimé en termes de position de ligne (angle de Bragg 2 thêta, exprimé en degrés ±0,2°) et de distances interplanaires d (exprimées en Å) :
| N° de ligne | Angle 2-thêta (degrés) | Distance interplanaire (Å) |
|---|---|---|
| 1 | 5,55 | 15,93 |
| 2 | 5,62 | 15,73 |
| 3 | 6,62 | 13,36 |
| 4 | 7,39 | 11,95 |
| 5 | 10,17 | 8,70 |
| 6 | 11,49 | 7,70 |
| 7 | 11,83 | 7,48 |
| 8 | 16,01 | 5,53 |
| 9 | 16,54 | 5,36 |
| 10 | 17,04 | 5,20 |
| 11 | 18,98 | 4,67 |
| 12 | 19,18 | 4,63 |
| 13 | 21,90 | 4,06 |
| 14 | 22,28 | 3,99 |
| 15 | 24,89 | 3,58 |

6. Forme cristalline I du sel hydrogénosulfate du Composé A selon l'une quelconque des revendications 1 à 5, **caractérisée en ce qu'**elle présente un spectre ¹³C CP/MAS RMN à l'état solide présentant les pics suivants (exprimés en ppm ± 0,2 ppm) : 173,31 ppm, 155,32 ppm, 140,46 ppm, 139,19 ppm, 137,42 ppm, 134,68 ppm, 131,65 ppm, 131,14 ppm, 129,37 ppm, 126,32 ppm, 118,77 ppm, 117,36 ppm, 116,54 ppm, 113,61 ppm, 112,69 ppm, 110,74 ppm, 102,33 ppm, 101,45 ppm, 63,06 ppm, 57,19 ppm, 54,87 ppm, 52,06 ppm, 44,71 ppm, 43,94 ppm, 34,42 ppm, 32,89 ppm, 31,28 ppm, 30,66 ppm, 14,40 ppm, 13,34 ppm, 12,49 ppm et 10,50 ppm.

7. Composition pharmaceutique comprenant comme ingrédient actif le sel hydrogénosulfate du Composé A selon la revendication 1 en association avec un ou plusieurs excipients pharmaceutiquement acceptables.

8. Composition pharmaceutique comprenant comme ingrédient actif la forme cristalline I du sel hydrogénosulfate du Composé A selon l'une quelconque des revendications 2 à 6 en association avec un ou plusieurs excipients pharmaceutiquement acceptables.

9. Composition pharmaceutique selon la revendication 7 ou 8 destinée à être utilisée dans le traitement de cancers, de maladies auto-immunes et de maladies du système immunitaire.

10. Composition pharmaceutique destinée à être utilisée selon la revendication 9, dans laquelle le cancer est choisi parmi les cancers de la vessie, du cerveau, du sein et de l'utérus, les leucémies lymphoïdes chroniques, le cancer du côlon, les cancers de l'œsophage et du foie, les leucémies lymphoblastiques, la leucémie aiguë myéloïde, les lymphomes, par exemple le lymphome non hodgkinien à cellules B et le lymphome diffus à grandes cellules B, les mélanomes, les hémopathies malignes, par exemple le syndrome myélodysplasique, les myélomes, par exemple le myélome multiple, le cancer de l'ovaire, le cancer du poumon non à petites cellules, le cancer de la prostate, le cancer du pancréas et le cancer du poumon à petites cellules.

11. Sel hydrogénosulfate du Composé A selon la revendication 1 destiné à être utilisé comme médicament.

12. Forme cristalline I du sel hydrogénosulfate du Composé A selon l'une quelconque des revendications 2 à 6 destinée à être utilisée comme médicament.

13. Procédé de préparation de la forme cristalline I du sel hydrogénosulfate du Composé A selon l'une quelconque des revendications 2 à 6, dans lequel le sel hydrogénosulfate du Composé A est cristallisé dans un milieu polaire.

14. Procédé de préparation de la forme cristalline I du sel hydrogénosulfate du Composé A selon la revendication 13, dans lequel le milieu polaire est composé d'un ou plusieurs solvants choisis parmi l'eau et les alcools.

15. Procédé de préparation de la forme cristalline I du sel hydrogénosulfate du composé A selon la revendication 14, dans lequel l'alcool est l'éthanol.

16. Procédé de préparation de la forme cristalline I du sel hydrogénosulfate du composé A selon la revendication 14, dans lequel le milieu polaire est un mélange éthanol/eau.

17. Procédé de préparation de la forme cristalline I du sel hydrogénosulfate du Composé A selon l'une quelconque des revendications 13 à 16, dans lequel procédé la cristallisation est ensemencée à l'aide d'une très petite quantité de la forme cristalline I du sel hydrogénosulfate du Composé A.

18. Forme cristalline anhydre du sel hydrogénosulfate de 5-(5-chloro-2-{[(3*S*)-3-(morpholin-4-ylméthyl)-3,4-dihydroisoquinolin-2(1*H*)-yl]carbonyl}phényl)-*N*-(5-cyano-1,2-diméthyl-1*H*-pyrrol-3-yl)-*N*-(4-hydroxyphényl)-1,2-diméthyl-1*H-*pyrrole-3-carboxamide (Composé A, H₂SO₄) selon la revendication 1, dans laquelle la forme cristalline présente un diagramme de diffraction des rayons X sur poudre présentant au moins 4, 5, 6, 7, 8, 9, 10, 11, 12, 13, 14, 15 ou l'ensemble des raies de diffraction suivantes (angle de Bragg 2 thêta, exprimé en degrés ± 0,2°) : 5,19 ; 5,64 ; 6,74 ; 7,14 ; 8,04 ; 8,33 ; 9,17 ; 9,40 ; 10,68 ; 11,03 ; 11,35 ; 12,18 ; 12,59 ; 13,64 ; 14,78 ; 15,09.

19. Forme cristalline anhydre du sel hydrogénosulfate du Composé A selon la revendication 18, **caractérisée en ce qu'**elle présente le diagramme de diffraction des rayons X sur poudre suivant, mesuré à l'aide d'un diffractomètre PANalytical X'Pert Pro MPD avec un détecteur X'Celerator et exprimé en termes de position de ligne (angle de Bragg 2 thêta, exprimé en degrés ±0,2°) et de distances interplanaires d (exprimées en Å) :
| N° de ligne | Angle 2-thêta (degrés) | Distance interplanaire (Å) |
|---|---|---|
| 1 | 5,19 | 17,03 |
| 2 | 5,64 | 15,66 |
| 3 | 6,74 | 13,12 |
| 4 | 7,14 | 12,39 |
| 5 | 8,04 | 10,99 |
| 6 | 8,33 | 10,61 |
| 7 | 9,17 | 9,64 |
| 8 | 9,40 | 9,41 |
| 9 | 10,68 | 8,29 |
| 10 | 11,03 | 8,02 |
| | | |
|---|---|---|
| 11 | 11,35 | 7,79 |
| 12 | 12,18 | 7,26 |
| 13 | 12,59 | 7,03 |
| 14 | 13,64 | 6,49 |
| 15 | 14,78 | 5,99 |
| 16 | 15,09 | 5,87 |
